# EUROPEAN PATENT SPECIFICATION

(11) **EP 3 353 524 B1**
(45) Date of publication and mention of the grant of the patent: **04.11.2020**
(21) Application number: 16892854.7
(22) Date of filing: 29.02.2016
(51) Int. Cl.: G01N 1/34, G01N 33/48

(54) **ENZYMATIC SAMPLE PURIFICATION**
AUFREINIGUNG ENZYMATISCHER PROBEN
PURIFICATION D'ÉCHANTILLON ENZYMATIQUE

(43) Date of publication of application: 01.08.2018
(73) Proprietor: Hewlett-Packard Development Company, L.P., Spring TX 77389 (US)
(72) Inventor: SHKOLNIKOV, Viktor, Palo Alto, California 94304 (US); ROGACS, Anita, Palo Alto, California 94304 (US)
(74) Representative: Haseltine Lake Kempner LLP
(86) International application number: PCT/US2016/020129
(87) International publication number: WO 2017/151103

(56) References cited:
- EP-A1- 0 370 163
- WO-A2-2004/077017
- US-A- 5 705 813
- US-A1- 2004 115 785
- US-A1- 2004 180 415
- US-A1- 2011 250 167
- US-A1- 2012 129 150
- US-A1- 2012 321 518
- W Kallow ET AL: "Penicillin biosynthesis: energy requirement for tripeptide precursor formation by delta-(L-alpha-aminoadipyl)-L-cysteinyl-D- valine synthetase from Acremonium chrysogenum", Biochemistry, 28 April 1998 (1998-04-28), page 5947, XP055556325, United States Retrieved from the Internet: URL:https://pubs.acs.org/doi/pdf/10.1021/b i971741o

## Description

### BACKGROUND

Complex Samples, such as blood, serum, cell lysate, extracellular fluid, milk and other biological fluids are often analyzed to identify analytes within the samples or to characterize analytes within the samples. Identifying or characterizing the analytes amongst the multiple molecules of such complex samples is often difficult and time-consuming.

### BRIEF DESCRIPTION OF THE DRAWINGS

Figure 1 is a schematic diagram of an example enzymatic sample purification system.
Figure 2 is a flow diagram of an example enzymatic sample purification method.
Figure 3 is a schematic diagram illustrating an example enzymatic sample purification method.
Figure 4 is a schematic diagram of an example enzymatic sample purification system.
Figure 5 is a schematic diagram illustrating an example enzymatic sample purification method.
Figure 6 is a schematic diagram of an example enzymatic sample purification system.

### DETAILED DESCRIPTION OF EXAMPLES

Disclosed herein are different examples of enzymatic sample purification systems and methods that utilize enzymes to facilitate faster and less costly preparation of a sample for analysis. Many complex samples such as blood, serum, cell lysate, extracellular fluid, milk and other biological fluids contain degradation products of protein, such as amino acids and peptides, which interfere with the detection and sensing of target analytes in the complex sample. Because the protein degradation products may have sizes and other properties similar to that of many target analytes, separation and removal of such protein degradation products may be difficult. The example enzymatic sample purification systems and methods utilize enzymes that react with protein degradation products to form much larger sized polypeptides. The example enzymatic sample purification systems and methods utilize differences between the target analyte and the much larger sized polypeptides to separate out the larger sized polypeptides, leaving a purified sample of the target analyte for analysis.

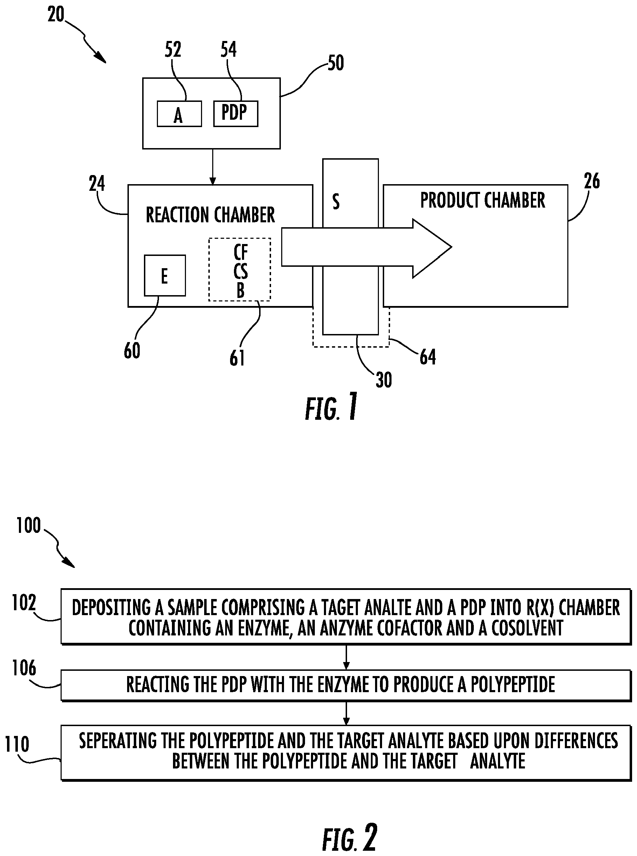

Figure 1 schematically illustrates an example enzymatic sample purification system 20 which may facilitate faster, less costly and more effective purification of a sample from what for analysis. System 20 purifies the sample by utilizing enzymes that react with protein degradation products to form much larger sized polypeptides, wherein system 20 separates out the much larger sized polypeptides from the much smaller target analyte to form a purified sample. As shown by Figure 1, system 20 comprises reaction chamber 24, product chamber 26 and separator (S) 30.

Reaction chamber 24 comprise a chamber having a port 34 to receive a sample 50 containing a target analyte (A) 52 and a protein degradation product (PDP) 54 (schematically shown). Reaction chamber 24 contains a polypeptide synthesizing enzyme 60. Enzyme 60 is specifically chosen based upon the characteristics of the target analyte 52 in sample 50 to be analyzed. Enzyme 60 specifically chosen so as to react with PDP 54 to formed polypeptides while not reacting with analyte 52 to formed polypeptides. Examples of different protein synthesizing enzymes 60 include, but are not limited to, proteases such as pepsin, papain, trypsin, elastase and others. Depending upon the target analyte 60, other polypeptide synthesizing enzymes 60 may be utilized.

As illustrated by broken lines in Figure 1, in some implementations, reaction chamber 24 may contain other elements such as enzyme cofactors (CF), cosolvents (CF) and buffers (B) 61. Enzyme cofactors are nonprotein molecules that catalyze reactions between the enzymes and the PDP 54. Examples of enzyme cofactors include, but are not limited to, cupric ions.

Cosolvents comprise liquid molecules that suspend enzyme 60 and dissolve other substances to form a solution. In one implementation, the cosolvents may be chosen to enhance the reactivity of enzyme 60 with PDP 54. In one implementation, the cosolvents may be organic. In other implementations, cosolvent may be aqueous. Examples of cosolvents include, but are not limited to, glycerol.

Buffers comprise molecules that, as part of the solution, inhibit changes to the pH of the solution. Such buffers may serve as a means of keeping pH at a nearly constant value. Examples of buffers include, but are not limited to, phosphate buffer. Each of the enzyme cofactors, cosolvents, and buffers may be chosen to facilitate thermodynamic conditions favorable for the formation, rather than the breakage of amide bonds to form the polypeptides.

In one implementation, reaction chamber 24 is prefilled with the enzyme 60 as well as enzyme cofactors, cosolvents and buffers, wherein reaction chamber 24 is then "factory sealed" until use. In one implementation, system 20 may be labeled for use with a particular type of sample 50, a particular type of PDP 54 and/or a particular type of target analyte 52, wherein the reaction chamber 24 is prefilled with the appropriate amount and relative amounts of a selected enzyme or multiple enzymes 60 as well as respective cofactors, cosolvents and buffers most suitable for the particular type of sample 50, the particular type of PDP 54 and/or the particular type of target analyte 52. As a result, different systems 20 with different combinations prefilled solution mixes within reaction chamber 24 may be specifically tuned for the exclusion of particular PDPs 54, particular amino acids and/or oligopepetides. Because system 20 may be mass-produced under controlled manufacturing specifications, the task of individual preparing of the enzymatic "cocktail" may be reduced in scope or eliminated, potentially reducing technician error. Moreover, the time and cost associated with individually preparing a solution to purify sample 50 is reduced. Because system 20 is self-contained, system 20 may be adapted for portable applications.

Product chamber 26 comprises a chamber that receives the target analyte 52 after the target analyte 52 has been separated from PDP 54 in the sample 50 by size-based separator (SBS) 30. In one implementation, product chamber 26 is connected to reaction chamber 24, directly or indirectly. In one implementation, product chamber 26 is indirectly connected reaction chamber 24, wherein SBS 30 extends between and is connected to on opposite sides to chambers 24 and 26. In some implementations, product chamber 26 may contain a chemical solution selected to preserve or otherwise further prepare the extracted target analyte 52 for analysis.

Separator 30 comprises a structure or device that separates the target analyte 52 with respect to the polypeptides resulting from the reaction of enzyme 60 with PDP 54 in reaction chamber 24. In one implementation, separator 30 may comprise a structure or device that separates target analyte 52 from the formed polypeptides based upon size differences between the molecules of target analyte 52 and the molecules of the formed polypeptides. In one implementation, separator 30 may comprise any of a variety of different size-based separators selected from a group of size-based separators consisting of a size exclusion chromatography separator, a dialysis separator, an electro-dialysis separator, and an electrophoresis separator. In other implementations, separator 30 may utilize other size-based separation technology.

In another implementation, separator 30 may comprise a structure into which the solution containing the analyte 52 and the polypeptides formed from the reaction of enzyme 60 and PDP 54 flow. In one implementation, the reaction chamber 24 may comprise a first capture moiety while separator 30 comprises and/or fixedly supports a second capture moiety, wherein the two capture moieties become bound to one another. The first capture moiety contained within reaction chamber 24 is chosen so as to become incorporated into the formed peptides. As a result, the formed peptides combined to the second capture moiety of the separator 30, inhibiting the flow of the polypeptides into out of separator 30, while permitting target analyte 52 to be discharged from separator 30 and into product chamber 26.

In one implementation, reaction chamber 24 comprises a first capture moiety such as an amino acid histidine. Separator 30 comprises a porous matrix or other membrane having an immobilized nickel, wherein the amino acid histidine nine becomes incorporated into the formed polypeptides and binds to the nickel of the porous matrix.

In one implementation, separator 30 is integrally formed as a single unitary body with reaction chamber 24 and product chamber 26, permanently fixed between chambers 24 and 26. For example, one implementation, separator 30 may comprise a membrane or filter which allows molecules of a certain size to pass therethrough, wherein the membrane or filter allows the smaller sized target analyte molecules 52 to pass from chamber 24, across separator 30, into product chamber 26, while the larger sized formed polypeptides (and PDP 54) are inhibited from passing through separator 30 into reaction chamber 24.

In yet another implementation, separator 30 may comprise a removable unit or component of system 20. For purposes of this disclosure, the term "releasably" or "removably" with respect to an attachment or coupling of two structures means that the two structures may be repeatedly connected and disconnected to and from one another without material damage to either of the two structures or their functioning. For example, as indicated by broken lines in 1, in one implementation, chambers 24 and 26 may be interconnected by a channel, slot or other receiver 64 that removably receives SBS 30 between chambers 24 and 26. In such an implementation, receiver 64 facilitates the removal and exchange of separator 30, facilitating reuse of system 20 when separator 30 is no longer usable and/or facilitating the modification of system 20 to accommodate the purification of different samples 50. In one implementation, receiver 64 facilitates the swapping of different separators 30, wherein the different separators 30 may have different filtering characteristics or size-based permeability depending upon the anticipated sizes of the target analyte 52 and the polypeptides resulting from the reaction of enzyme 60 and PDP 54. For example, smaller sized target analyte 52 and larger sized target analyte 52 may be better purified from the respective samples utilizing different SBSs 30. In another implementation, receiver 64 facilitates the swapping of different separators 30, wherein the different separators 30 may have different capture moieties that cooperate with the corresponding capture moieties in reaction chamber 24 that become incorporated into the formed polypeptides.

In yet other implementations, separator 30 may comprise a separate component or device, distinct from one or both of chambers 24 and 26. For example, in other implementations, separator 30 may be provided as part of a single unit with chamber 24, wherein the target analyte 52 is extracted through separator 30 into a separate product chamber 26.

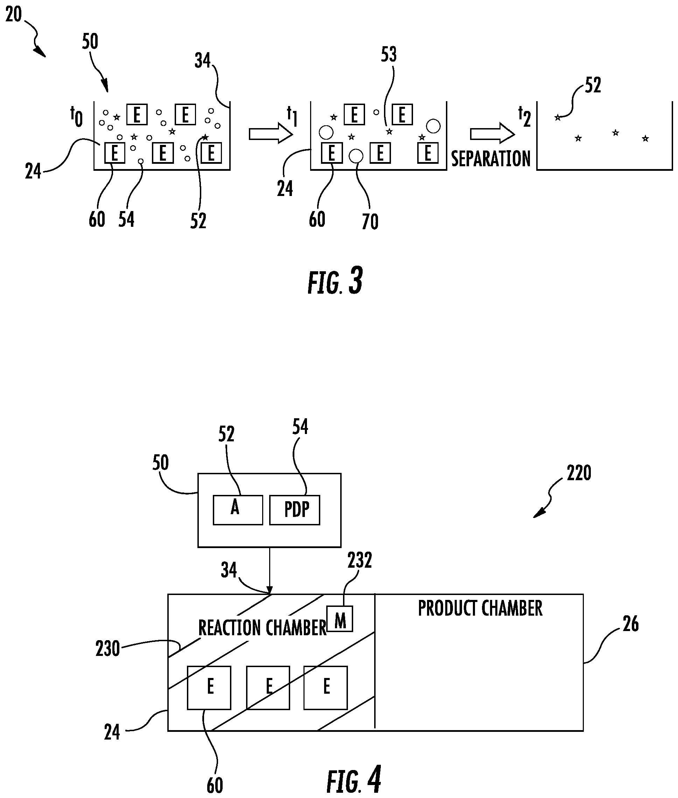

Figures 2 and 3 illustrate an example method 100 for enzymatically purifying a sample to prepare the sample for analysis. Although method 100 is described as being carried out by system 20, method 100 may alternatively be carried out by any of the other enzymatic sample purification systems described hereafter or the other appropriate enzymatic sample purification systems. As indicated by block 102 and illustrated at time t₀ in Figure 3, sample 50 is deposited through port 34 into reaction chamber 24. Sample 50 comprises target analyte 52 (schematically illustrated with stars) and PDP 54 (schematically illustrated as an amino acid with solid circles). Examples of sample 50 include, but are not limited to, blood, serum, cell lysate, extracellular fluid, milk and/or other biological fluids. Target analyte 52 may comprise small molecules that are unable to form peptide bonds (amide bonds). Such markets do not have primary and mine groups or carboxylic acid groups and are therefore, unable to form amide bonds.

Analyte 52 may additionally or alternatively comprise molecules that are able to form peptide bonds, but do not form peptide bonds under the enzymatic conditions present in reaction chamber 24 due to the particular enzyme 60 chosen or the other conditions of reaction chamber 24. Such molecules may have primary amine groups or carboxylic acid groups, but are unable to undergo peptide bond formation given the particular enzyme 60 within reaction chamber 24. Such an analyte may include amino acids or a oligopeptides that are sterically excluded from the active site of the peptide forming enzyme. Examples of such analytes include, but are not limited to, biomarkers such as thyrotropin-releasing hormone (a tripeptide), Leu-enkephalin, Met-enkephalin (pentapeptides), and others. In one method, the analyte 52 and PDP 54 of sample 50, prior to purification, have molecular weights and radii of gyration that overlap or that are similar to make separation by size alone difficult. In addition, in some implementations, the molecules of analyte 52 and the molecules of PDP 54 may also have similar charge, electrophoretic mobility, partition coefficient (related to hydrophobicity), protein kinase A (pKa) properties, rendering ion selective, electrophoretic and/or liquid and solid-based separation separation difficult.

As further indicated by block 102, reaction chamber 24, into which sample 50 is deposited, contains enzymes (E) 60, an enzyme cofactor and a cosolvent. In one implementation, reaction chamber 24 additionally contains a buffer. Enzyme 60, the enzyme cofactor, the cosolvent and the buffer are each described above.

As indicated by block 106 and illustrated at time t₂, in Figure 3, the enzyme 60 and the PDP 54 are allowed to react with one another to form polypeptides 70 (schematically illustrated with open circles). In one implementation, this reaction is allowed to go to completion, reaching an equilibrium state, prior to separation in block 110. In other implementations, separation in block 110 is carried out prior to the reaction reaching completion, wherein the ongoing reactions within chamber 24 are terminated for separation.

As indicated by block 110 and illustrated at time t₃ in Figure 3, the target analyte 52 is then separated from the formed or created polypeptides by separator 30 based upon the differences between polypeptides 70 and target analyte 52. In one implementation, the target analyte 52 is separated from the polypeptides based upon size differences between the molecules of analyte 52 and the polypeptides. In one implementation, the polypeptides 70 each have a size much greater than the size of the molecules of target analyte 52. In one implementation, the polypeptides 70 or at least 100 percent larger than target analyte 52. In one implementation, polypeptides 70 have a size of at least 1000 Da of the target analyte has a size of no greater than 200 Da.

In the example illustrated, the separated out target analyte 52 is transported to product chamber 26. As a result, the target analyte 52 may be better detected and analyzed, with less interference from any remaining PDP 54. As described above, in some implementations, separator 30 may comprise a selectively permeable membrane or filter, wherein the target analyte 52 is allowed to pass through the membrane or filter into product chamber 26 while the larger polypeptides 70 (and any remaining enzymes 60) remain captured within reaction chamber 24. In other implementations other separation techniques may be employed.

Figure 4 schematically illustrates an example enzymatic sample purification system 220, an example implementation of system 20. As with system 20, system 220 may facilitate faster, less costly and more effective purification of a sample to the sample for analysis. System 220 purifies the sample by utilizing enzymes that react with protein degradation products to form much larger sized polypeptides, wherein system 220 separates out the much larger sized polypeptides from the much smaller target analyte to form a purified sample. System 220 is similar to system 20 described above except that system 220 additionally comprises porous matrix 230 which replaces and serves as a separator. Those remaining components of system 220 which correspond to components of system 220 are numbered similarly. Although not shown, reaction chamber 24 of system 220 may additionally comprise enzyme cofactors, cosolvents and a buffer 61 as illustrated and described with respect to Figure 2.

Porous matrix 230 comprises a grid or matrix of a material or multiple materials disposed within and contained within reaction chamber 24. Porous matrix 30 comprises a matrix of interconnected internal spaces or volumes, referred to as cells or pores. In one implementation, the open celled pores of matrix 30 are sized larger than the size of the molecules of target analyte 52, but smaller than the size of the to be formed polypeptides, resulting from the reaction of enzyme 60 and PDP 54. In the example illustrated, porous matrix 230 entropically traps enzymes 60 within reaction chamber 24.

In one implementation, porous matrix 230 is formed from or fixedly carries a first capture moiety selected to facilitate binding with a second capture moiety that incorporates into or formed as part of the to be formed polypeptides. In such an implementation, the formed polypeptides are more likely to become captured or immobilized onto matrix 230 as a result of the moieties incorporated into the formed polypeptides. For example, in one implementation, moiety 232 may comprise amino acid histidine while matrix 230 is formed from or carries an immobilized nickel such that the formed polypeptides bind to a nickel on the matrix 230. In other implementations, other combinations of capture moieties, in the solution within reaction chamber 24 and upon matrix 230, may be employed. In some implementations, the separation of the formed polypeptides and the target analyte 52 is facilitated using both size-based separation as described above and the binding between the capture moieties in the polypeptides and the capture moieties in the porous matrix 230.

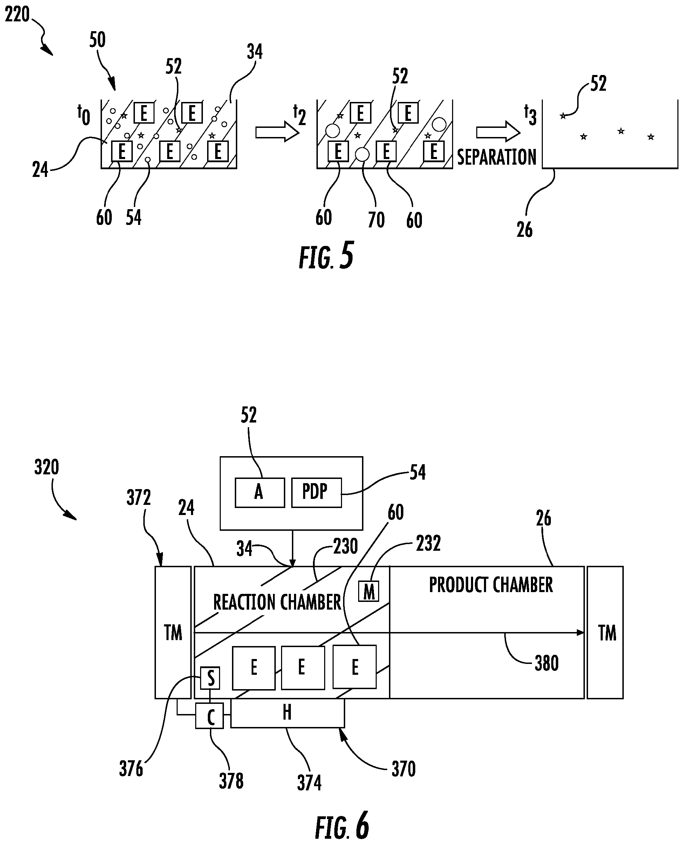

Figure 5 is a diagram schematically illustrating the purification of a sample 50 by system 220. As illustrated at time t₀ in Figure 5, sample 50 is deposited through port 34 into reaction chamber 24. Sample 50 comprises target analyte 52 (schematically illustrated with stars) and PDP 54 (schematically illustrated as an amino acid with solid circles). Examples of sample 50 include, but are not limited to, blood, serum, cell lysate, extracellular fluid, milk and/or other biological fluids. Target analyte 52 may comprise small molecules that are unable to form peptide bonds (amide bonds). Such molecules do not have primary amine groups or carboxylic acid groups and are therefore, unable to form amide bonds.

Analyte 52 may additionally or alternatively comprise molecules that are able to form peptide bonds, but do not form peptide bonds under the enzymatic conditions present in reaction chamber 24 due to the particular enzyme 60 chosen or the other conditions of reaction chamber 24. Such molecules may have primary amine groups or carboxylic acid groups, but are unable to undergo peptide bond formation given the particular enzyme 60 within reaction chamber 24. Such an analyte may include amino acids or a oligopeptides that are sterically excluded from the active site of the peptide forming enzyme. Examples of such analytes include, but are not limited to, biomarkers such as thyrotropin-releasing hormone (a tripeptide), Leu-enkephalin, Met-enkephalin (pentapeptides), and others. In one method, the analyte 52 and PDP 54 of sample 50, prior to purification, have molecular weights and radii of gyration that overlap one another or that are similar to one another to make separation by size alone difficult. In addition, in some implementations, the molecules of analyte 52 and the molecules of PDP 54 may also have similar charge, electrophoretic mobility, partition coefficient (related to hydrophobicity), protein kinase A (pKa) properties, rendering ion selective, electrophoretic and/or liquid and solid-based separation separation difficult.

As further illustrated by Figure 5, reaction chamber 24, into which sample 50 is deposited, contains enzymes (E) 60, an enzyme cofactor and a cosolvent. In one implementation, reaction chamber 24 additionally contains a buffer. Enzyme 60, the enzyme cofactor, the cosolvent and the buffer are each described above. The enzyme 60 may be captured or retained within porous matrix 230

As illustrated at time t₂, in Figure 5, the enzyme 60 and the PDP 54 are allowed to react with one another to form polypeptides 70 (schematically illustrated with open circles). In one implementation, this reaction is allowed to go to completion, reaching an equilibrium state, prior to separation. In other implementations, separation is carried out prior to the reaction reaching completion, wherein the ongoing reactions within chamber 24 are terminated for separation.

As illustrated at time t₃ in Figure 5, the target analyte 52 is then separated from the formed or created polypeptides by porous matrix 230 based upon the differences between polypeptides 70 and target analyte 52. In one implementation, the target analyte 52 is separated from the polypeptides based upon size differences between the molecules of analyte 52 and the polypeptides. In one implementation, the polypeptides 70 each have a size much greater than the size of the molecules of target analyte 52. In one implementation, the polypeptides 70 or at least 100 percent larger than target analyte 52. In one implementation, polypeptides 70 have a size of at least 1000 of the target analyte has a size of no greater than 200.

In the example illustrated, the separated out target analyte 52 is transported to product chamber 26. As a result, the target analyte 52 may be better detected and analyzed, with less interference from any remaining PDP 54. In one implementation, reaction chamber 24 and the contained polypeptides and target analyte 52 are centrifuged, whereby the polypeptides remain captured within porous matrix 230 while the target analyte 52 is discharged from reaction chamber 230. In other implementations other separation techniques may be employed.

Figure 6 schematically illustrates an example enzymatic sample purification system 320. System 320 is similar to system 220 described above except that system 320 additionally comprises temperature control system 370 and transport mechanism 372. Components or structures of system 320 which correspond to structures of systems 20 or 220 are numbered similarly.

Temperature control system 370 provides closed-loop feedback for the control of the temperature of the solution within reaction chamber 24 to facilitate enzymatic reactions between enzymes 60 and the PDP 54 of the received sample 50 within reaction chamber 24. Temperature control system 370 comprises heater 374, sensor 376 and controller 378. Heater 374 comprises a device that generates and emits heat so as to heat the contents of reaction chamber 24. In one implementation, heater 374 comprises an electrically conductive material which produces heat through resistance to electrical current. In another implementation, heater 374 may comprise other forms of heat generating and emitting devices.

Sensor 376 comprises a device that senses a temperature of the solution within reaction chamber 24. Controller 378 comprises a processing unit that controls the operation of heater 374 based upon signals received from sensor 376 and the sensed temperature of the content of reaction chamber 24. By controlling the temperature of the solution within reaction chamber 24, the formation of peptides through the reaction of enzymes 60 with PDP 54 may be enhanced.

For purposes of this application, the term "processing unit" shall mean electronics or hardware that executes sequences of instructions contained in a non-transitory memory. Execution of the sequences of instructions causes the processing unit to perform steps such as generating control signals. The instructions may be loaded in a random access memory (RAM) for execution by the processing unit from a read only memory (ROM), a mass storage device, or some other persistent storage. In other embodiments, hard wired circuitry may be used in place of or in combination with software instructions to implement the functions described. For example, controller 378 may be embodied as part of one or more application-specific integrated circuits (ASICs). Unless otherwise specifically noted, the controller is not limited to any specific combination of hardware circuitry and software, nor to any particular source for the instructions executed by the processing unit.

Transport mechanism 372 comprises a device that moves purified portions of sample 50 from reaction chamber 24 into product chamber 26. In one implementation, transport mechanism 372 comprises a pressurized liquid flow transport, wherein pressurized liquid is applied to reaction chamber 24 to move solution within reaction chamber 24 into product chamber 26 in the direction indicated by arrow 380. In another implementation, transport mechanism 372 comprises an electrophoretic transport which applies an electric field in the direction indicated by arrow 382 buys movement of the solution of sample 50, including target analyte 52, towards product chamber 26. In both implementations, the target analyte 52 is moved product chamber 26 while the polypeptides formed from the reaction of enzymes 60 and PDP 54 remain captured within reaction chamber 24 by porous matrix 230.

In one implementation, controller 378 further controls the operation of transport mechanism 372. In one implementation, controller 378 controls the time at which transport mechanism 372 is activated. By controlling the time at which transport mechanism 372 is activated or the rate at which transport mechanism 372 operates, controller 378 may control the size of the formed polypeptides at the time that the sample 50 is driven into product chamber 26. By controlling the time of the reaction as well as the temperature in other properties of the solution in which the reactions occur, the length and size of the formed polypeptides may be controlled. As a result, controller 378 may control the temperature of reaction chamber 24 and the activation of transport mechanism 372 at a time when the formed polypeptides have a sufficient length and are sufficiently sized such that the formed polypeptides have a size or other properties so as to remain captured within the porous matrix 230 when the liquid flow bias is applied by transport mechanism 372.

Although the present disclosure has been described with reference to example implementations, workers skilled in the art will recognize that changes may be made in form and detail without departing from the spirit and scope of the claimed subject matter. For example, although different example implementations may have been described as including one or more features providing one or more benefits, it is contemplated that the described features may be interchanged with one another or alternatively be combined with one another in the described example implementations or in other alternative implementations. Because the technology of the present disclosure is relatively complex, not all changes in the technology are foreseeable. The present disclosure described with reference to the example implementations and set forth in the following claims is manifestly intended to be as broad as possible. For example, unless specifically otherwise noted, the claims reciting a single particular element also encompass a plurality of such particular elements. The terms "first", "second", "third" and so on in the claims merely distinguish different elements and, unless otherwise stated, are not to be specifically associated with a particular order or particular numbering of elements in the disclosure.

## Claims

1. An apparatus comprising:
a reaction chamber (24) containing a polypeptide synthesizing enzyme, the reaction chamber (24) having a port (34) to receive a sample comprising a target analyte and a protein degradation product;
a product chamber (26); and
a separator (30) configured to retain polypeptides resulting from reaction of the protein degradation product with the polypeptide synthesizing enzyme in the reaction chamber (24) and to transport purified remaining portions of the sample comprising a target analyte to the product chamber (26).

2. The apparatus of claim 1 further comprising a porous matrix (230) within the reaction chamber (24) immobilizing the polypeptide synthesizing enzymes.

3. The apparatus of claim 1, wherein the separator (30) comprises:
a porous matrix (230) within the reaction chamber (24), the porous matrix (230) configured to immobilize the polypeptide; and
a transport mechanism (372) configured to move the purified portions of the sample to the product chamber (26).

4. The apparatus of claim 3, wherein the transport mechanism (372) is selected from a group of transport mechanisms consisting of an electrophoretic transport and a pressurized liquid flow transport.

5. The apparatus of claim 1, wherein the separator (30) is selected from a group of separators consisting of: a size exclusion chromatography separator, a dialysis separator, an electro-dialysis separator, and an electrophoresis separator.

6. The apparatus of claim 1 further comprising a receiver (64) interconnected to the reaction chamber (24) and the product chamber (26), the receiver (64) sized to removably receive and retain the separator (30) between the reaction chamber (24) and the product chamber (26).

7. The apparatus of claim 1 further comprising:
a first capture moiety in the reaction chamber (24) to be incorporated into the formed polypeptides; and
a second capture moiety carried by the separator (30), the second capture moiety to bind with the first capture moiety of the formed polypeptides.

8. A method (100) comprising:
depositing (102) a sample comprising a target analyte and a protein degradation product into a reaction chamber (24) containing a polypeptide synthesizing enzyme, to form a solution in the reaction chamber (24);
reacting (106) the protein degradation product with the polypeptide synthesizing enzyme to produce a polypeptide; and
separating (110) the polypeptide and the target analyte;
wherein the polypeptide synthesizing enzyme is specifically chosen so as to react with the protein degradation product while not reacting with the target analyte.

9. The method of claim 8, wherein separating the polypeptide and the target analyte comprise retaining the polypeptide within the reaction chamber (24) and transporting the target analyte to a separate product chamber (26).

10. The method of claim 9, wherein the retention of the polypeptide within the reaction chamber (24) is with a porous matrix (230) within the reaction chamber (24).

11. The method of claim 8, wherein separating the polypeptide and the target analyte comprising applying an electric field to the solution, wherein the electric field moves the polypeptide and the target analyte at different rates in response to the size differences between the polypeptide and the target analyte.

12. The method of claim 8 further comprising:
sensing a temperature of the reaction chamber (24);
activating a heater (374) to apply heat to the reaction chamber based upon the sensed temperature of the reaction chamber (24).

13. The method of claim 8 further comprising preventing the target analyte from forming peptide bonds.

14. The method of claim 8, wherein the target analyte and the protein degradation product have overlapping molecular sizes.

15. An enzymatic sample purifier comprising:
a reaction chamber (24) containing a polypeptide synthesizing enzyme, the reaction chamber (24) having a port (34) to receive a sample comprising a protein degradation product and a target analyte; and
a porous matrix (230) within the reaction chamber (24), the porous matrix configured to retain polypeptides resulting from reaction of the protein degradation product with the polypeptide synthesizing enzyme in the reaction chamber (24), without retaining the target analyte.

## Patentansprüche

1. Vorrichtung, die Folgendes umfasst:
eine Reaktionskammer (24), die ein Polypeptidsynthese-Enzym enthält, wobei die Reaktionskammer (24) eine Öffnung (34) zur Aufnahme einer Probe aufweist, die einen Zielanalyten und ein Proteinabbauprodukt umfasst;
eine Produktkammer (26); und
einen Separator (30), der dazu konfiguriert ist, Polypeptide zurückzuhalten, die aus der Reaktion des Proteinabbauprodukts mit dem Polypeptidsynthese-Enzym in der Reaktionskammer (24) resultieren, und gereinigte verbleibende Abschnitte der Probe, die einen Zielanalyten umfasst, zu der Produktkammer (26) zu transportieren.

2. Vorrichtung nach Anspruch 1, die ferner eine poröse Matrix (230) innerhalb der Reaktionskammer (24) umfasst, die die Polypeptid-Synthese-Enzyme immobilisiert.

3. Vorrichtung nach Anspruch 1, wobei der Separator (30) Folgendes umfasst:
eine poröse Matrix (230) innerhalb der Reaktionskammer (24), wobei die poröse Matrix (230) dazu konfiguriert ist, das Polypeptid zu immobilisieren; und
einen Transportmechanismus (372), der dazu konfiguriert ist, die gereinigten Abschnitte der Probe zu der Produktkammer (26) zu bewegen.

4. Vorrichtung nach Anspruch 3, wobei der Transportmechanismus (372) aus einer Gruppe von Transportmechanismen ausgewählt ist, die aus Folgenden besteht: einem elektrophoretischen Transport und einem unter Druck stehenden Flüssigkeitsströmungstransport.

5. Vorrichtung nach Anspruch 1, wobei der Separator (30) aus einer Gruppe von Separatoren ausgewählt ist, die aus Folgenden besteht: einem Größenausschlusschromatographieseparator, einem Dialyseseparator, einem Elektrodialyseseparator und einem Elektrophoreseseparator.

6. Vorrichtung nach Anspruch 1, die ferner einen Empfänger (64), der mit der Reaktionskammer (24) und der Produktkammer (26) verbunden ist, umfasst, wobei der Empfänger (64) bemessen ist, um den Separator (30) zwischen der Reaktionskammer (24) und der Produktkammer (26) entfernbar aufzunehmen und zu halten.

7. Vorrichtung nach Anspruch 1, die ferner Folgendes umfasst:
einen ersten Einfanganteil in der Reaktionskammer (24), der in die ausgebildeten Polypeptide eingebaut werden soll; und
einen zweiten Einfanganteil, der durch den Separator (30) getragen wird, wobei sich der zweite Einfanganteil mit dem ersten Einfanganteil der ausgebildeten Polypeptide binden soll.

8. Verfahren (100), das Folgendes umfasst:
Ablagern (102) einer Probe, die einen Zielanalyten und ein Proteinabbauprodukt umfasst, innerhalb einer Reaktionskammer (24), die ein Polypeptidsynthese-Enzym enthält, um eine Lösung in der Reaktionskammer (24) auszubilden;
Umsetzen (106) des Proteinabbauprodukts mit dem Polypeptid-Synthese-Enzym, um ein Polypeptid herzustellen; und
Trennen (110) des Polypeptids und des Zielanalyten;
wobei das Polypeptid-Synthese-Enzym speziell ausgewählt ist, um mit dem Proteinabbauprodukt zu reagieren, ohne mit dem Zielanalyten zu reagieren.

9. Verfahren nach Anspruch 8, wobei das Trennen des Polypeptids und des Zielanalyten ein Zurückhalten des Polypeptids innerhalb der Reaktionskammer (24) und ein Transportieren des Zielanalyten zu einer separaten Produktkammer (26) umfasst.

10. Verfahren nach Anspruch 9, wobei die Rückhaltung des Polypeptids innerhalb der Reaktionskammer (24) mit einer porösen Matrix (230) innerhalb der Reaktionskammer (24) erfolgt.

11. Verfahren nach Anspruch 8, wobei das Trennen des Polypeptids und des Zielanalyten ein Anlegen eines elektrischen Feldes an die Lösung umfasst, wobei das elektrische Feld das Polypeptid und den Zielanalyten als Reaktion auf die Größenunterschiede zwischen dem Polypeptid und dem Zielanalyten mit unterschiedlichen Geschwindigkeiten bewegt.

12. Verfahren nach Anspruch 8, das ferner Folgendes umfasst:
Erfassen einer Temperatur der Reaktionskammer (24);
Aktivieren einer Heizung (374), um Wärme an die Reaktionskammer auf der Basis der erfassten Temperatur der Reaktionskammer (24) anzulegen.

13. Verfahren nach Anspruch 8, das ferner ein Verhindern umfasst, dass der Zielanalyt Peptidbindungen ausbildet.

14. Verfahren nach Anspruch 8, wobei der Zielanalyt und das Proteinabbauprodukt überlappende Molekülgrößen aufweisen.

15. Enzymatischer Probenreiniger, der Folgendes umfasst:
eine Reaktionskammer (24), die ein Polypeptidsynthese-Enzym enthält, wobei die Reaktionskammer (24) eine Öffnung (34) zum Aufnehmen einer Probe aufweist, die ein Proteinabbauprodukt und einen Zielanalyten aufweist; und
eine poröse Matrix (230) innerhalb der Reaktionskammer (24), wobei die poröse Matrix dazu konfiguriert ist, Polypeptide zurückzuhalten, die aus der Reaktion des Proteinabbauprodukts mit dem Polypeptid-Synthese-Enzym in der Reaktionskammer (24) resultieren, ohne den Zielanalyten zurückzuhalten.

## Revendications

1. Appareil comprenant :
une chambre de réaction (24) contenant une enzyme de synthèse de polypeptide, la chambre de réaction (24) comportant un orifice (34) permettant de recevoir un échantillon comprenant un analyte cible et un produit de dégradation de protéine ;
une chambre de produit (26) ; et
un séparateur (30) conçu pour retenir des polypeptides résultant de la réaction du produit de dégradation de protéine avec l'enzyme de synthèse de polypeptide dans la chambre de réaction (24) et pour transporter des parties restantes purifiées de l'échantillon comprenant un analyte cible vers la chambre de produit (26).

2. Appareil selon la revendication 1, comprenant en outre une matrice poreuse (230) à l'intérieur de la chambre de réaction (24) immobilisant les enzymes de synthèse de polypeptide.

3. Appareil selon la revendication 1, dans lequel le séparateur (30) comprend :
une matrice poreuse (230) à l'intérieur de la chambre de réaction (24), la matrice poreuse (230) étant conçue pour immobiliser le polypeptide ; et
un mécanisme de transport (372) conçu pour déplacer les parties purifiées de l'échantillon vers la chambre de produit (26).

4. Appareil selon la revendication 3, dans lequel le mécanisme de transport (372) est choisi parmi un groupe de mécanismes de transport constitué d'un transport par électrophorèse et d'un transport d'écoulement de liquide sous pression.

5. Appareil selon la revendication 1, dans lequel le séparateur (30) est choisi dans un groupe de séparateurs constitué : d'un séparateur de chromatographie d'exclusion de taille, d'un séparateur de dialyse, d'un séparateur d'électrodialyse et d'un séparateur d'électrophorèse.

6. Appareil selon la revendication 1, comprenant en outre un récepteur (64) interrelié à la chambre de réaction (24) et à la chambre de produit (26), le récepteur (64) étant dimensionné pour recevoir et retenir de manière amovible le séparateur (30) entre la chambre de réaction (24) et la chambre de produit (26).

7. Appareil selon la revendication 1, comprenant en outre :
un premier fragment de capture dans la chambre de réaction (24) à incorporer dans les polypeptides formés ; et
un second fragment de capture porté par le séparateur (30), le second fragment de capture étant destiné à se lier au premier fragment de capture des polypeptides formés.

8. Procédé (100) comprenant :
le dépôt (102) d'un échantillon comprenant un analyte cible et un produit de dégradation de protéine dans une chambre de réaction (24) contenant une enzyme de synthèse de polypeptide, pour former une solution dans la chambre de réaction (24) ;
la mise en réaction (106) du produit de dégradation de protéine avec l'enzyme de synthèse de polypeptide pour produire un polypeptide ; et
la séparation (110) du polypeptide et de l'analyte cible ;
dans lequel l'enzyme de synthèse de polypeptide est spécifiquement choisie de manière à réagir avec le produit de dégradation de protéine sans réagir avec l'analyte cible.

9. Procédé selon la revendication 8, dans lequel la séparation du polypeptide et de l'analyte cible comprend la rétention du polypeptide à l'intérieur de la chambre de réaction (24) et le transport de l'analyte cible vers une chambre de produit (26) séparée.

10. Procédé selon la revendication 9, dans lequel la rétention du polypeptide à l'intérieur de la chambre de réaction (24) est avec une matrice poreuse (230) à l'intérieur de la chambre de réaction (24).

11. Procédé selon la revendication 8, dans lequel la séparation du polypeptide et de l'analyte cible comprend l'application d'un champ électrique à la solution, dans lequel le champ électrique déplace le polypeptide et l'analyte cible à des vitesses différentes en réponse aux différences de taille entre le polypeptide et l'analyte cible.

12. Procédé selon la revendication 8, comprenant en outre :
la détection d'une température de la chambre de réaction (24) ;
l'activation d'un élément chauffant (374) pour appliquer de la chaleur à la chambre de réaction sur la base de la température détectée de la chambre de réaction (24).

13. Procédé selon la revendication 8, comprenant en outre le fait d'empêcher l'analyte cible de former des liaisons peptidiques.

14. Procédé selon la revendication 8, dans lequel l'analyte cible et le produit de dégradation de protéine ont des tailles moléculaires qui se chevauchent.

15. Purificateur d'échantillons enzymatique comprenant :
une chambre de réaction (24) contenant une enzyme de synthèse de polypeptide, la chambre de réaction (24) comportant un orifice (34) permettant de recevoir un échantillon comprenant un produit de dégradation de protéine et un analyte cible ; et
une matrice poreuse (230) à l'intérieur de la chambre de réaction (24), la matrice poreuse étant conçue pour retenir des polypeptides résultant de la réaction du produit de dégradation de protéine avec l'enzyme de synthèse de polypeptide dans la chambre de réaction (24), sans retenir l'analyte cible.
